# EUROPEAN PATENT APPLICATION

(11) **EP 4 692 831 A1**
(43) Date of publication of application: **11.02.2026**
(21) Application number: 24193872.9
(22) Date of filing: 09.08.2024
(51) Int. Cl.: G01R 33/34

(54) **MAGNETIC RESONANCE IMAGING BREAST COIL ASSEMBLY AND RELATED METHOD**

(71) Applicant: Koninklijke Philips N.V., 5656 AG Eindhoven (NL)
(72) Inventor: AHMAD, Rizwan, 5656 AG Eindhoven (NL); RAGHURAMAN, Sairamesh, 5656 AG Eindhoven (NL); BHAWSAR, Rajat, 5656 AG Eindhoven (NL); RAMESH, Bollimuntha, 5656 AG Eindhoven (NL); K V, Raghavendiran, 5656 AG Eindhoven (NL)
(74) Representative: Philips Intellectual Property & Standards

(57) **Abstract**

Disclosed herein is a magnetic resonance breast coil assembly (100, 200) comprising a breast specific medial support (104). The breast specific medial support comprises a first cupped surface (106) configured for providing medial support and vertical support to a breast (102). The magnetic resonance breast coil assembly further comprises a breast specific tiltable flap (108). The breast specific tiltable flap comprises a second cupped surface (110) configured for providing lateral support and vertical support to the breast. The breast specific tiltable flap has an adjustable tilt (608, 700) configured to at least partially stabilize the breast by tilting to hold the breast between the first cupped surface and the second cupped surface. The magnetic resonance breast coil assembly further comprises at least one breast specific magnetic resonance imaging coil (402, 404, 406, 408).

## Description

### FIELD OF THE INVENTION

The invention relates to magnetic resonance imaging, in particular to magnetic resonance imaging coils for breast screening.

### BACKGROUND OF THE INVENTION

Although digital mammography (DM) has become an accepted standard of care in screening and diagnosis of breast cancer, up to 30% of breast cancers are not detected by standard screening. This percentage is even higher in women with dense breasts. Hence, risk of breast cancer is increased, and cancers may be masked and missed on mammography due to superposition of tissue; as a result, there might be an excess of late-stage disease.

Also, Mammography is a painful process to go through. Patients will have to stand in front of a special X-ray machine. A technologist will place the breast on a plastic plate. Another plate will firmly press the breast from above. The plates will flatten the breast, holding it still while the X-ray is being taken. The steps are repeated to make a side view of the breast. The other breast will be X-rayed in the same way. Having a mammogram is uncomfortable and painful for most women. The squeezing of breasts between plastic plates creates pressure, discomfort, and pain.

United States patent application publication US20170143204A discloses an MR imaging system for breast tissue. The MR imaging system includes a pair of antenna coil arrays that are movable with respect to a base plate on which the coil arrays are mounted. A fixed coil array may also be mounted in a medial location on the base plate. The MR imaging system provides an abdominal support structure and a head support structure to position a patient in a prone position over the movable and fixed coil arrays. An additional support pad may be provided on the upper surface of the medial coil to support the patient's chest. Each of the movable antenna coil arrays may be moved laterally along the base plate to adjust the space between the two arrays. Once adjusted, each of the movable antenna coil arrays may be secured in the adjusted position to immobilize the breast tissue.

### SUMMARY OF THE INVENTION

In one aspect a magnetic resonance breast coil assembly is disclosed. The magnetic resonance breast coil assembly comprises a breast specific medial support. The breast specific medial support comprises a first cupped surface configured to provide medial support and vertical support to a breast. The magnetic resonance breast coil assembly further comprises a breast-specific tiltable flap. The breast-specific tiltable flap comprises a second cupped surface configured for providing lateral support and a vertical support to the breast. The breast-specific tiltable flap has an adjustable tilt configured to at least partially stabilize the breast by tilting to hold the breast between the first cupped surface and the second cupped surface. The magnetic resonance breast coil assembly further comprises at least one breast-specific magnetic resonance imaging coil.

In another aspect, a magnetic resonance imaging system comprises the magnetic resonance breast coil assembly according to an embodiment is disclosed.

In another aspect, a method of magnetic resonance imaging using the magnetic resonance breast coil assembly is disclosed. The method comprises stabilizing the breast by the breast-specific adjustable tiltable flap with the first cupped surface and the second cupped surface. The method further comprises acquiring, by magnetic resonance imaging system, k-space data using the at least one breast-specific magnetic resonance imaging coil. The method further comprises reconstructing a magnetic resonance image using the k-space data.

### BRIEF DESCRIPTION OF THE DRAWINGS

In the following preferred embodiments of the invention will be described, by way of example only, and with reference to the drawings in which:
Fig. 1 illustrates an example of a magnetic resonance breast coil assembly.
Fig. 2 illustrates a further example of a magnetic resonance breast coil assembly.
Fig. 3 shows a further view of the magnetic resonance breast coil assembly of Fig. 2.
Fig. 4 shows a further view of the magnetic resonance breast coil assembly of Fig. 1 or 2.
Fig. 5 illustrates the implementation of a cylindrical cam in the magnetic resonance breast coil assembly of Fig. 1 or 2.
Fig. 6 illustrates the implementation of a ratchet assembly in the magnetic resonance breast coil assembly of Fig. 1 or 2.
Fig. 7 illustrates the implementation of a self-locking interference fit for the breast specific tiltable flap.
Fig. 8 illustrates the implementation of a tilt ratchet mechanism for the breast specific tiltable flap.
Fig. 9 illustrates the routing of radio-frequency cables through the pivot shaft of the breast specific tiltable flap.
Fig. 10 illustrates a magnetic resonance imaging system incorporating the magnetic resonance breast coil assembly of Fig. 1 or 2.

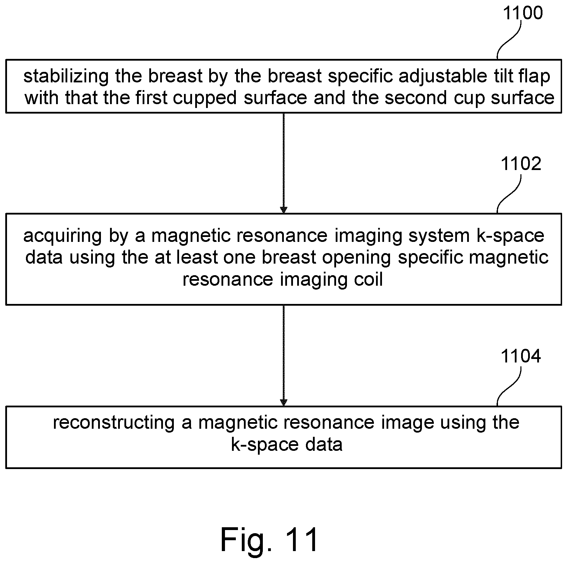

Fig. 11 shows a flow chart which illustrates a method of operating the magnetic resonance imaging system of Fig. 10.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

In one example, there is a magnetic resonance breast coil assembly. The magnetic resonance breast coil assembly comprises a breast specific medial support. The breast specific medial support comprises a first cupped surface configured for providing medial support and vertical support to a breast. The magnetic resonance breast coil assembly further comprises a breast-specific tiltable flap. The breast-specific tiltable flap comprises a second cupped surface configured for providing lateral support and vertical support to the breast. The breast-specific tiltable flap has an adjustable tilt configured to at least partially stabilize the breast by tilting to hold the breast between the first cupped surface and the second cupped surface. The magnetic resonance breast coil assembly further comprises at least one breast-specific magnetic resonance imaging coil. This example may be beneficial because it may provide for a more comfortable means of supporting a breast during a magnetic resonance imaging examination. Current breast supports work by essentially crushing the breast between two supports that squeeze it. This is particularly uncomfortable and for medical modalities such as magnetic resonance imaging it is difficult for the subject to remain still long enough to obtain magnetic resonance images without motion artifacts. The two-cupped surfaces are used to support the breast instead of crushing it. This may not only result in a more comfortable examination for the subject but, due to reduced motion of the subject, may result in higher quality magnetic resonance images.

As used herein, the term medial is used to refer to something that is closer or nearer to the mid-line of the body. The breast specific medial support is therefore closer to the mid-line of the subject and the breast-specific tiltable flap is further to the medial region of the subject.

The references to breast-specific, as used herein, may also refer to breast opening specific. Often times, when breast coils are used in magnetic resonance imaging, the subject reposes in a prone position with the subject laying flat with the chest down and the back up. The breasts may then hang and be supported by the breast specific medial support and the breast-specific tiltable flap.

In the above example and in subsequent examples, reference is made to one breast and also to breast specific medial support and a single-breast-specific tiltable flap. It is understood herein that the claims and description are also applicable to a pair of such supports.

Examples may have the benefit of being able to support and immobilize the breasts during a magnetic resonance imaging examination without compressing or pressing the breasts. For example, US20170143204A discloses replacing an x-ray machine with a magnetic resonance imaging system. However, the antenna arrays are only movable in a lateral fashion. They crush and compress the breasts in the same way that an x-ray mammography system would which could result in discomfort and pain during the magnetic resonance imaging examination.

In another example, the breast-specific tiltable flap is configured for tilting away from the breast specific medial support to provide an access port to the breast. The tiltable flap is used to tilt towards the medial direction to hold the breast between the first cupped surface and the second cupped surface. However, when a subject is lying prone and is using the magnetic resonance breast coil assembly there is no access to the breast normally. For example, if the breast is in a bad position or needs to be repositioned, it may be inconvenient or impossible and the subject may have to be removed and then again positioned. An advantage of this example is that the tiltable flap can tilt away from the medial direction providing an access port to the breast, which enables the breast of the subject to be adjusted while the subject is in a prone position. This may be more comfortable for the subject and may result in a faster magnetic resonance examination.

In another example, the adjustable tilt is implemented by supporting the breast specific tiltable flap from a pivot shaft with a self-locking interference fit. For example, the pivot shaft may be formed with two shafts and then rubber or other material used to add an interference fit can be used so that once the breast-specific tiltable flap is tilted in the proper position it does not move during the duration of a magnetic resonance imaging examination. This may be beneficial because it may provide for an extremely simple but effective mechanism for implementing this.

In another example, the adjustable tilt is implemented by supporting the breast-specific tiltable flap from a pivot shaft with a ratchet mechanism. For example, the breast-specific tiltable flap may be designed such that it can be tilted increasingly towards the medial direction and then may be releasable. This means that the flap can be put into increments until the breast is properly supported. This may provide for a very stable way of supporting the breast during a magnetic resonance imaging examination.

In another example, the magnetic resonance breast coil assembly further comprises radio-frequency cables routed through the pivot shaft for connecting to a portion of the at least one breast-specific magnetic resonance imaging coil. This may be beneficial because it may reduce or eliminate cable slack during the magnetic resonance imaging examination. This may for example result in a better signal-to-noise ratio in the resulting magnetic resonance image.

In another example, the at least one breast-specific magnetic resonance imaging coil comprises one or more medial breast surface coils located adjacent to the first cupped surface. Being adjacent to the first cupped surface may mean that the surface coils are positioned near the surface of the breast and may result in very good quality magnetic resonance images.

In another example, the at least one breast specific magnetic resonance imaging coil comprises at least two medial breast surface coils located adjacent to the first cupped surface. In another example, the at least one breast specific magnetic resonance imaging coil comprises at least four medial breast surface coils located adjacent to the first cupped surface. Increasing the number of coils enables the use of parallel imaging techniques which may be used to accelerate the acquisition of k-space data during imaging of the breasts.

In another example, the one or more medial breast surface coils are located on or attached to the first cupped surface. Being located on or attached to the first cupped surface may result in better quality magnetic resonance images.

In another example, the at least one breast-specific magnetic resonance imaging coil comprises one or more lateral breast surface coils located adjacent to the second cupped surface. The placement of the one or more lateral breast surface coils adjacent to the second cupped surface may have the benefit that this enables the ability to acquire k-space data close to the breast during a parallel imaging magnetic resonance imaging protocol.

In another example, the at least one breast-specific magnetic resonance imaging coil comprises at least two lateral breast surface coils located adjacent to the second cupped surface. In another example, the at least one breast-specific magnetic resonance imaging coil comprises at least four lateral breast surface coils located adjacent to the second cupped surface.

In another example, the one or more lateral breast surface coils are located on or attached to the second cupped surface. This for example brings the one or more lateral breast surface coils closer to the breast and may result in a higher quality magnetic resonance image.

In another example, the at least one breast-specific magnetic resonance imaging coil comprises at least one anterior surface coil. The at least one anterior surface coil is preferably configured for imaging an axial region of the subject. This example is particularly beneficial because during breast cancer, cancer cells may metastasize to lymph nodes in the axial region. By being able to image both the breast and the axial region at the same time, this may result in a higher quality examination for the subject and may also reduce the time as only one examination is needed instead of two.

In another example, the at least one breast-specific magnetic resonance imaging coil comprises at least one posterior surface coil. The at least one posterior surface coil is preferably distal to the breast when the subject is in the magnetic resonance breast coil assembly. This may for example have the benefit of providing for better imaging of the breast.

In another example, the breast-specific tiltable flap is configured for a translational motion in a lateral direction. For example, the breast-specific tiltable flap can be slid in a medial direction towards the breast specific medial support. This may have the example that the positioning of the first and second surface with respect to each other can also be positioned in a lateral direction. This may result in better support of the breast during the magnetic resonance imaging protocol.

In another example, the translational motion is controlled by using a releasable ratchet or a cylindrical cam. Both examples provide for a means of positioning the breast-specific tiltable flap laterally and locking it into place.

In another example, the magnetic resonance breast coil assembly further comprises a subject support configured for supporting a subject in a horizontal or prone position. The subject support comprises at least one breast opening for receiving the breast of the subject. It may also receive both breasts to two different breast openings.

In another example, the subject support is formed from a flexible surface supported by a frame. This may be beneficial because having the flexible surface may enable it to better conform to the subject and therefore be more comfortable.

In another example, the flexible surface is formed from a textile. The use of a textile may be beneficial because this may be magnetic resonance imaging compatible as well as comfortable for the subject being imaged.

In another example, the subject support is configured such that a chest region of the subject is supported horizontally and the pelvic region of the subject is inclined below the chest region.

In another example, the subject support is configured such that the subject is supported in a prone position.

In another aspect, a magnetic resonance imaging system comprising the magnetic resonance breast coil assembly of an example is disclosed. The incorporation of the magnetic resonance breast coil assembly into a magnetic resonance imaging system may be beneficial because the subject will be held in a more comfortable and sustainable manner than using traditional breast supports.

In another aspect, a method of magnetic resonance imaging using an example of the magnetic resonance breast coil assembly is disclosed. The method comprises stabilizing the breast by the breast-specific tiltable flap with the first cupped surface and the second cupped surface. The method further comprises acquiring using a magnetic resonance imaging system k-space data using the at least one breast-specific magnetic resonance imaging coil and the method further comprises reconstructing the magnetic resonance image using the k-space data. The use of the magnetic resonance breast coil assembly may have the benefit, as was mentioned above, that it is more comfortable and does not crush the breast of the subject. Due to the higher level of subject comfort and reduced chance of movement the magnetic resonance image may have fewer motion artifacts for example.

In another example, the breast specific tiltable flap is configured for tilting away from the breast specific medial support to provide an access port to the breast. The method further comprises adjusting the breast specific tiltable flap to provide the access port to the breast. The method further comprises adjusting a position of the breast before adjusting the adjustable tilt such that the first cupped surface and the second cupped surface stabilize the breast. This example may be very beneficial because it enables the adjustment of the subject's breast or breasts when the subject is in a prone position and is already mounted to the magnetic resonance breast coil assembly.

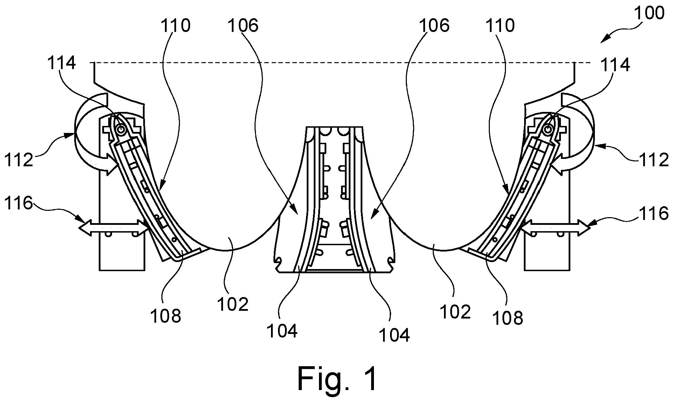

Fig. 1 illustrates an example of a magnetic resonance breast coil assembly 100. Visible are two breasts 102. In the middle there are two breast specific medial supports 104, one for each breast 102. Each of the breast-specific medial supports 104 has a first cupped surface 106. The first cupped surface 106 provides medial support and vertical support to the breast 102. Opposing each first cupped surface 106 is a breast-specific tiltable flap 108. The breast-specific tiltable flap 108 has a second cupped surface 110. The first cupped surface 106 and the second cupped surface 110 are able to immobilize the breast 102 during a magnetic resonance imaging procedure without crushing or squeezing the breast. The breast-specific tiltable flap 108 can be rotated 112 about a pivot shaft 114. By rotating the positions of the second cupped surface 110 can be adjusted such that, in conjunction with the first cupped surface 106, the breasts 102 are adequately immobilized during the entire magnetic resonance imaging procedure.

Additional to the rotational motion 112, the breast-specific tiltable flaps 108 are also able to move in a lateral direction 116 to better adjust the positioning of the second cupped surface 110 relative to the first cupped surface 106. In Fig. 1, the at least one breast-specific magnetic resonance imaging coil is not shown.

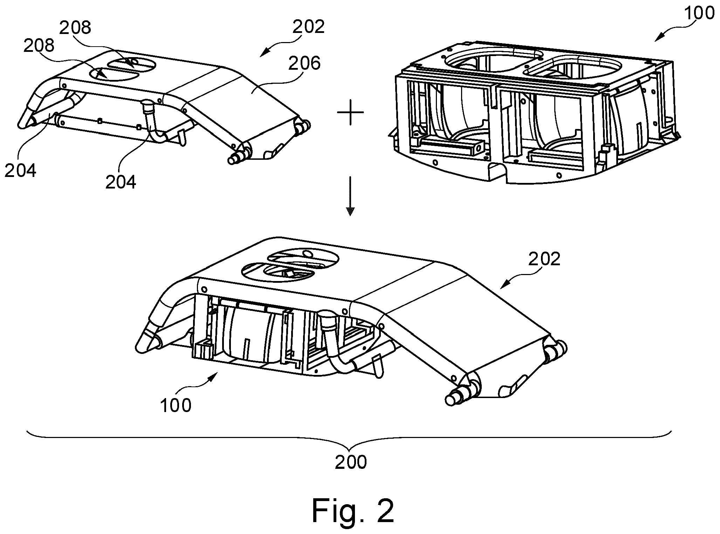

Fig. 2 illustrates a further example of the magnetic resonance breast coil assembly 200. In this case it is shown as comprising the magnetic resonance breast coil assembly 100, as was illustrated in Fig. 1, and additionally a subject support 202. The subject support 202 comprises a frame 204 with a flexible surface 206. When the subject is lying horizontal or prone on the subject support 202, the breasts are able to fit through the breast openings 208. Below, the magnetic resonance breast coil assembly 100 of Fig. 1 and the subject support 202 are shown as being assembled into an integral unit.

It can be seen that the subject support 202 is structured such that when a subject is lying prone with the breasts going through the breast openings 208, the pelvic region of the subject will be supported lower than the chest region. The design of the subject support 202 is hammock-like and adjusts to the specific shape of the subject automatically. The combination of this hammock-like frame 204 and the use of the breast-specific tiltable flap 108 results in a magnetic resonance breast coil assembly 200 that is significantly more comfortable than traditional magnetic resonance coil systems for performing mammography.

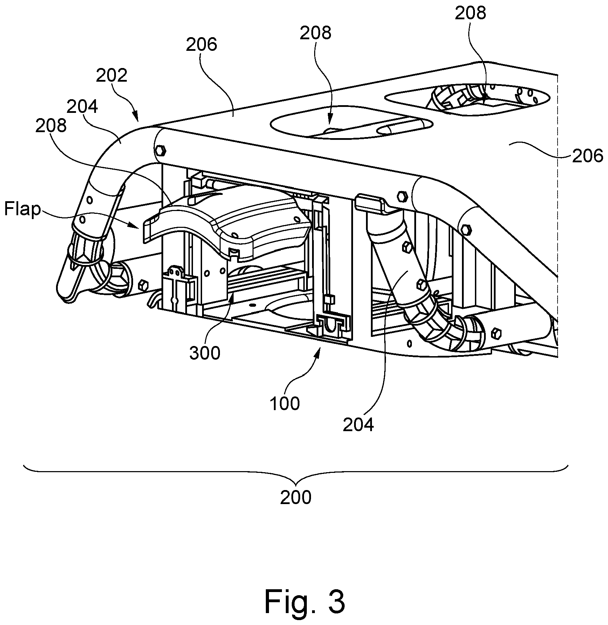

Fig. 3 shows a further view of the magnetic resonance breast coil assembly 200 of Fig. 2. The tiltable flap 108 has been rotated away from the breast-specific medial support 104. Doing this opens an access port 300, which enables adjustment of the breast position when a subject is being supported by the subject support 202. This provides a means of adjusting the position of the subject within the magnetic resonance breast coil assembly 100 without having to remove the subject. This may result in a more comfortable positioning of the subject before the examination as well as saving a large amount of time during the preparation and positioning of the subject for a magnetic resonance imaging examination.

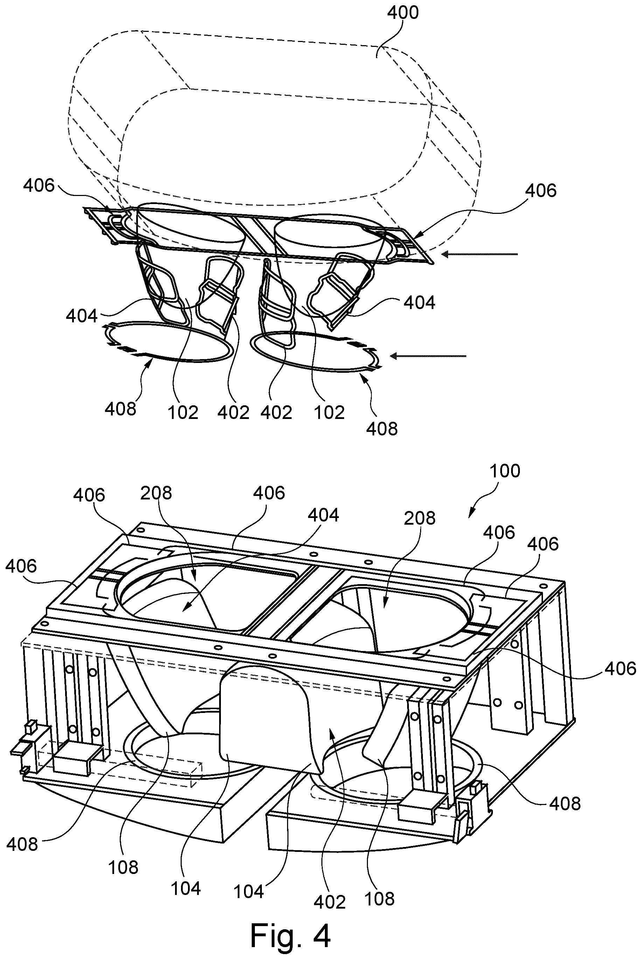

Fig. 4 illustrates the breast-specific magnetic resonance imaging coils. Two views are shown. Above, a view of a subject 400 with two breasts 102 is shown only above the breast-specific magnetic resonance imaging coils. At a medial position there are medial breast surface coils 402 adjacent to each breast 102. Lateral to this, there are lateral breast surface coils 404 positioned next to each breast 102 also. In an anterior position, there are a number of anterior surface coils 406. The anterior surface coils 406 are significant as in they also enable imaging of the chest region and the axial region of the subject. This is particularly useful when trying to detect cancerous tissue that may have metastasized to lymph nodes, particularly in the axial or armpit region. Positioned near the distal position of the breast 102 are a set of posterior surface coils 408 for each breast 102. The combination of surface coils 402, 404, 406, and 408 illustrated, illustrate that the large number of surface coils will enable parallel imaging procedures to enable the completely image the breast 102 as well as surrounding chest regions of the subject 400.

Below this view of the subject 400 is a view which shows the position of the coils 402, 404, 406, and 408 relative to the magnetic resonance breast coil assembly 100, as was illustrated in Fig. 1. The anterior surface coils 406 are shown as being mounted near the entrance to the breast opening 208. Opposing this at the base of the magnetic resonance breast coil assembly 100 is mounted the posterior surface coils 408. Although they are not visible in this Fig. the lateral breast surface coils 404 are mounted on or adjacent to the second curved surface of the breast-specific tiltable flaps 108. Likewise, adjacent to or just beneath the surface of the breast-specific medial supports 104 is the medial breast surface coils 402.

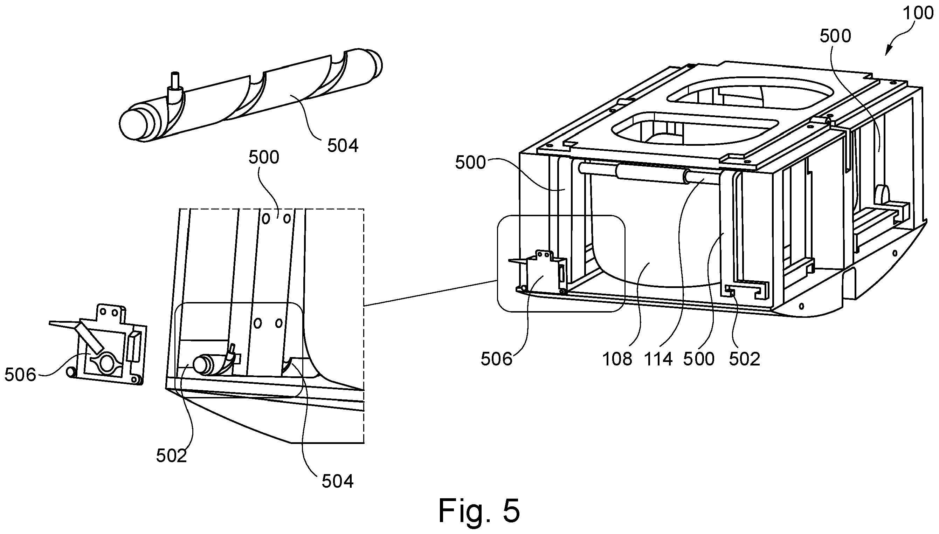

Fig. 5 illustrates one means of adding the lateral motion 116 to the breast-specific tiltable flap 108. The breast-specific tiltable flap 108 is supported by a pivot shaft 114. The pivot shafts 114 are connected on either side to guides 500. The guides 500 then slide within guide rails 502 and are actuated by a cylindrical cam 504. In some examples, there may also be a locking mechanism 506 to prevent the cylindrical cam 504 from turning and to lock the lateral motion of the breast-specific tiltable flap 108 in a chosen position. The breast-specific tiltable flap 108 can be moved laterally 116 by rotating the cylindrical cam 504.

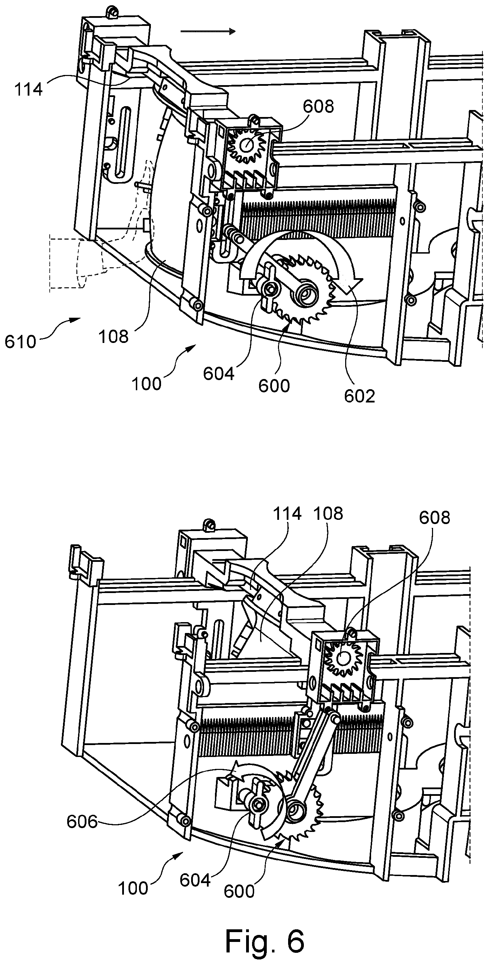

Fig. 6 shows an alternative for lateral motion 116 of the breast-specific tiltable flap 108. In this example, the position of the pivot shaft 114 is moved by a ratchet mechanism 600. A pressing motion 610 by the hand of an operator causes the breast-specific tiltable flap 108 to move and is held in place by the ratchet mechanism 600. To release this there is a release mechanism 604 which can be rotated 606 to release the ratchet 600 and allow the breast-specific tiltable flap 108 to be moved away from the medial position. A tilt ratchet mechanism 608 for controlling the tilt of the breast-specific tiltable flap 108 is also shown and is discussed in greater detail in a further Figure.

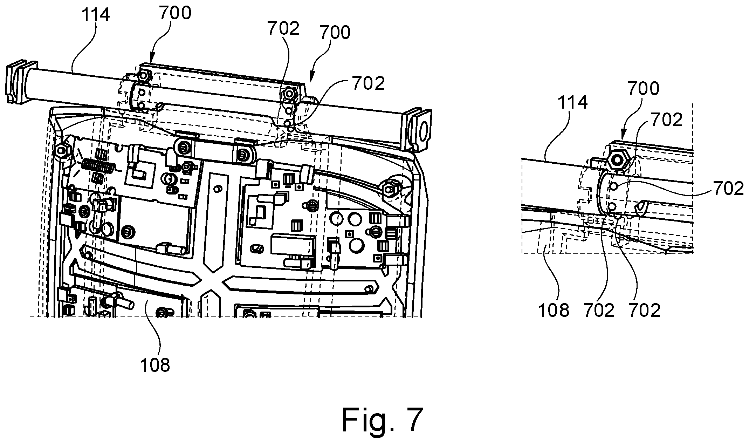

Fig. 7 illustrates the implementation of a self-locking interference fit 700. The pivot shaft 114 is used to support the breast-specific tiltable flap 108. At the contact points to the pivot shaft 114 there are multiple pivot interference projections 702. For example, these may be small rubber or elastic projections which are used to provide the self-locking interference fit.

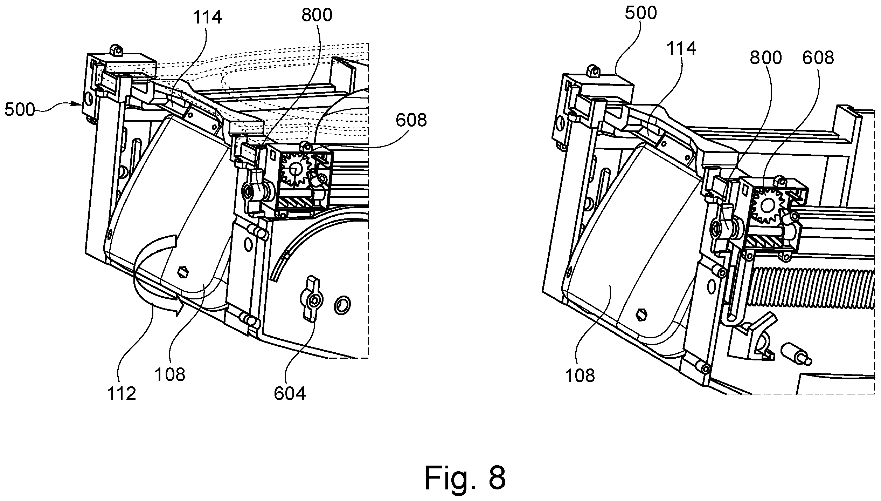

Fig. 8 shows a further view of the tilt ratchet mechanism 608. The tilt ratchet 608 is a ratchet mechanism which allows the breast-specific tiltable flap 108 to be moved or rotated 112 in the direction of the breast-specific medial support 104. The ratchet 608 however prevents the breast-specific tiltable flap 108 from rotating in the opposite direction. When it is desired to release the flap 108 a tilt ratchet release mechanism 800 may be actuated.

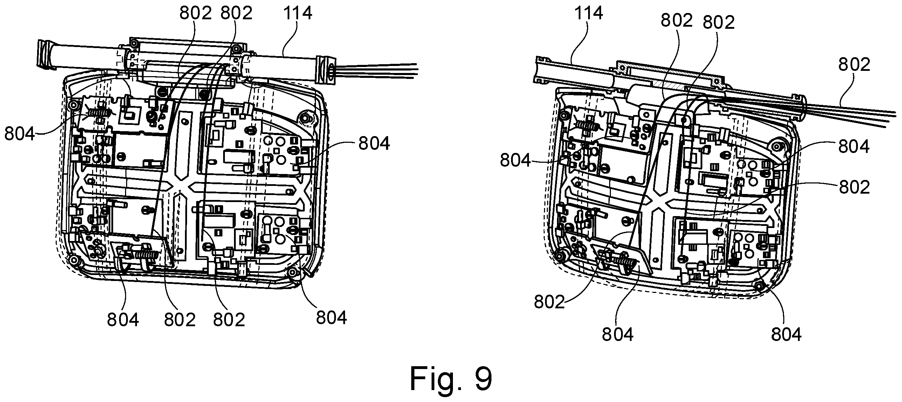

Fig. 9 illustrates the routing of radio-frequency cables 802 through the pivot shaft 114. The pivot shaft 114 is hollow and enables the radio-frequency cables 802 to be routed directly to the coil electronics 804. This prevents any slack in the radio-frequency cables 802 as well as keeping them safely away from the subject.

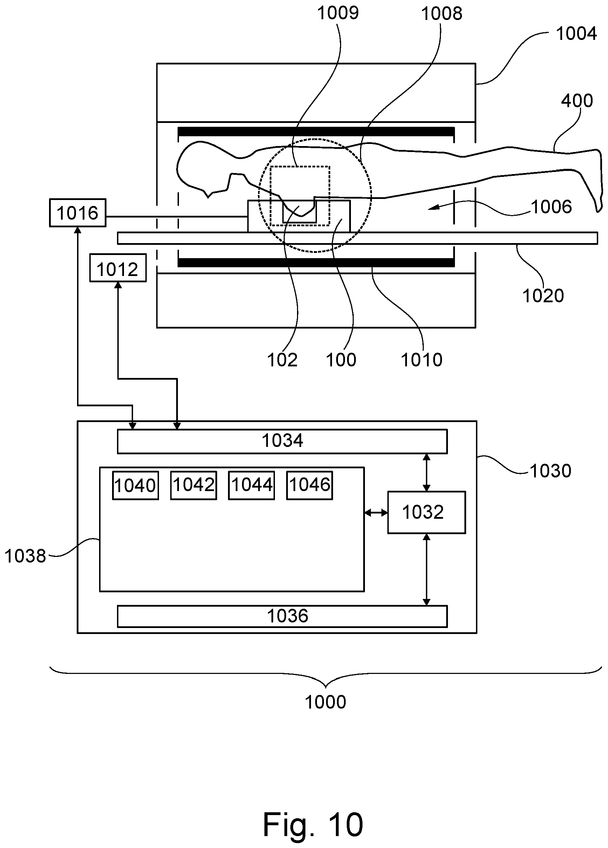

Fig. 10 illustrates an example of a magnetic resonance imaging system 1000 that incorporates the magnetic resonance breast coil assembly 100 of Fig. 1. The magnetic resonance breast coil assembly 200 that incorporates the subject support 202 may also be used.

The magnetic resonance imaging system 1000 comprises a magnet 1004. The magnet 1004 is a superconducting cylindrical type magnet with a bore 1006 through it. It is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject. The arrangement of the two sections area is similar to that of a Helmholtz coil. Open magnets are popular because the subject is less confined. Inside the cryostat of the cylindrical magnet there is a collection of superconducting coils.

Within the bore 1006 of the cylindrical magnet 1004 there is an imaging zone 1008 where the magnetic field is strong and uniform enough to perform magnetic resonance imaging. A field of view 1009 is shown within the imaging zone 1008. The k-space data are acquired for the field of view 1009. The region of interest could be identical with the field of view 1009 or it could be a sub volume of the field of view 1009. The subject 400 is shown as being supported by a subject support 1020 and the magnetic resonance breast coil assembly 100 such that the breasts 102 of the subject 400 are within the imaging zone 1008 and the field of view 1009.

Within the bore 1006 of the magnet there is also a set of magnetic field gradient coils 1010 which is used for the acquisition of measured k-space data to spatially encode magnetic spins within the imaging zone 1008 of the magnet 1004. The magnetic field gradient coils 1010 are connected to a magnetic field gradient coil power supply 1012. The magnetic field gradient coils 1010 are intended to be representative. Typically, magnetic field gradient coils 1010 contain three separate sets of coils for spatial encoding in three orthogonal spatial directions. A magnetic field gradient power supply supplies current to the magnetic field gradient coils. The current supplied to the magnetic field gradient coils 1010 is controlled as a function of time and may be ramped or pulsed.

Adjacent to the imaging zone 108 is the magnetic resonance breast coil assembly 100 for manipulating the orientations of magnetic spins within the imaging zone 1008 and for receiving radio transmissions from spins also within the imaging zone 1008. The magnetic resonance breast coil assembly 100 may contain multiple coil elements 402, 404, 406, 408. The magnetic resonance breast coil assembly 100 is connected to a radio frequency transceiver 1016. The magnetic resonance breast coil assembly 100 and radio frequency transceiver 1016 may be replaced by separate transmit and receive coils and a separate transmitter and receiver. The magnetic resonance breast coil assembly 100 may have multiple receive/transmit elements and the radio frequency transceiver 1016 may likewise have multiple receive/transmit channels. The transceiver 1016 and the gradient controller 1012 are shown as being connected to the hardware interface 1006 of a computer system 1002.

The computer 1030 is further shown as comprising a computational system 1032. The computer 1030 and the computational system 1032 may be one or more computer systems or computational systems located at one or more locations. The hardware interface 1034 may enable the computational system 1032 to communicate with and to control other components of the magnetic resonance imaging system 1000. The computational system 1032 is further shown as being in communication with an optional user interface 1036 and a memory 1038. The memory 138 is intended to represent different types of memory which may be accessible to the computational system 1032.

The memory 1038 is shown as containing machine-executable instructions 1040. The machine-executable instructions 140 enable the computational system 1032 to perform various data and image processing tasks as well as to control the magnetic resonance imaging system 1000. The memory 1038 is further shown as storing pulse sequence commands 1042. The pulse sequence commands 1042 are commands or data which may be converted into such commands for controlling the magnetic resonance imaging system 1000 to acquire k-space data. Typically, the pulse sequence commands 1042 are in the form of a timing diagram. The memory 1038 is further shown as storing a k-space data 1044 that was acquired by controlling the magnetic resonance imaging system 1000 with the pulse sequence commands 1042. The memory 1038 is further shown as storing a magnetic resonance image 1046 that was reconstructed from the k-space data 1044.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A 'user interface' may also be referred to as a `human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

Although the invention has been described in reference to specific embodiments, it should be understood that the invention is not limited to these examples only and that many variations of these embodiments may be readily envisioned by the skilled person after having read the present disclosure. The invention may thus further be described without limitation and by way of example only by the following embodiments. The following embodiments may contain preferred embodiments. Accordingly, the term "clause" as used therein may refer to such a "preferred embodiment".
Clause 1. A subject support configured for supporting a subject in a horizontal position, wherein the subject support further comprises at least one breast opening for receiving a breast of the subject, wherein the subject support is formed from a flexible surface supported by a frame;
   - at least one breast specific magnetic resonance imaging coil.
Clause 2. The subject support of clause 1, wherein the flexible surface is formed from a textile.
Clause 3. Subject support of clause 1 or 2, wherein the subject support is configured such that a chest region of the subject is supported horizontally and a pelvic region of the subject is inclined below the chest region.
Clause 4. A magnetic resonance breast coil assembly comprising:
   - the subject support of clause 1, 2, or 3;
   - a breast specific medial support, wherein the breast specific medial support comprises a first cupped shape surface configured for providing medial support and vertical support to the breast; and
   - a breast specific tiltable flap, wherein the breast specific tiltable flap support comprises a second cupped shape surface configured for providing lateral support and vertical support to the breast, wherein the breast specific tiltable flap comprises an adjustable tilt configured to at least partially stabilize the breast by tilting to hold the breast between the first cupped surface and the second cupped surface.
Clause 5. The magnetic resonance breast coil assembly of clause 4, wherein the breast specific tiltable flap is configured for tilting away from the breast specific medial support to provide an access port to the breast.
Clause 6. The magnetic resonance breast coil assembly of clause 4 or 5, wherein the adjustable tilt is implemented by supporting the breast specific tiltable flap from a pivot shaft with a self-locking interference fit.
Clause 7. The magnetic resonance breast coil assembly of clause 4 or 5, wherein the adjustable tilt is implemented by supporting the breast specific tiltable flap from a pivot shaft with a ratchet mechanism.
Clause 8. The magnetic resonance breast coil assembly of clause 6 or 7, wherein the magnetic resonance breast coil assembly further comprises radio frequency cable routed through the pivot shaft for connecting to a portion of the at least one breast specific magnetic resonance imaging coil.
Clause 9. The magnetic resonance breast coil assembly of any one of clauses 4 through 8, wherein the at least one breast specific magnetic resonance imaging coil comprises one or more medial breast surface coils located adjacent to the first cupped surface, wherein the at least one breast specific magnetic resonance imaging coil preferably comprises at least two medial breast surface coils located adjacent to the first cupped surface, wherein the at least one breast specific magnetic resonance imaging coil more preferably comprises at least four medial breast surface coils located adjacent to the first cupped surface.
Clause 10. The magnetic resonance breast coil assembly of clause 9, wherein the one or more medial breast surface coils are located on the first cupped surface.
Clause 11. The magnetic resonance breast coil assembly of any one of clauses 4 through 10, wherein the at least one breast specific magnetic resonance imaging coil comprises one or more lateral breast surface coils located adjacent to the second cupped surface, wherein the at least one breast specific magnetic resonance imaging coil preferably comprises at least two lateral breast surface coils located adjacent to the second cupped surface, wherein the at least one breast specific magnetic resonance imaging coil more preferably comprises at least four lateral breast surface coils located adjacent to the second cupped surface.
Clause 12. The magnetic resonance breast coil assembly of clause 11, wherein the one or more lateral breast surface coils are located on the second cupped surface.
Clause 13. The magnetic resonance breast coil assembly of any one of clauses 4 through 12, wherein the at least one breast specific magnetic resonance imaging coil comprises at least one anterior surface coil, and wherein the at least one anterior surface coil is preferably configured for imaging an axial region of the subject.
Clause 14. The magnetic resonance breast coil assembly of any one of clauses 4 through 13 wherein the at least one breast specific magnetic resonance imaging coil comprises at least one posterior surface coil, and wherein the at least one posterior surface coil is preferably distal to the breast.
Clause 15. The magnetic resonance breast coil assembly of any one of clauses 4 through 14, wherein the breast specific tiltable flap is configured for a translational motion in a lateral direction, wherein the translational motion is controlled using any one of the following: a releasable ratchet and a cylindrical cam.
Clause 16. A magnetic resonance imaging system comprising the magnetic resonance breast coil assembly of any one of clauses 1 through 15.

### REFERENCE SIGNS LIST

- 100: magnetic resonance breast coil assembly
- 102: breast
- 104: breast specific medial support
- 106: first cupped surface
- 108: breast specific tiltable flap
- 110: second cupped surface
- 112: rotational motion
- 114: pivot shaft
- 116: translational (lateral) motion
- 200: magnetic resonance breast coil assembly
- 202: subject support
- 204: frame
- 206: flexible surface
- 208: breast opening
- 300: access port
- 400: subject
- 402: medial breast surface coils
- 404: lateral breast surface coils
- 406: anterior surface coils
- 408: posterior surface coils
- 500: guide
- 502: guide rails
- 504: cylindrical cam
- 506: locking mechanism
- 600: ratchet mechanism for lateral motion
- 602: rotation of ratchet
- 604: release mechanism
- 606: motion to activate release mechanism
- 608: tilt ratchet mechanism
- 610: pressing motion
- 700: self locking interference fit
- 702: pivot interference projections
- 800: tilt ratchet release mechanism
- 802: radio frequency cables
- 804: coil electronics
- 1000: magnetic resonance imaging system
- 1004: magnet
- 1006: bore of magnet
- 1008: imaging zone
- 1009: field of view
- 1010: magnetic field gradient coils
- 1012: magnetic field gradient coil power supply
- 1016: transceiver
- 1020: subject support
- 1030: computer
- 1032: computational system
- 1034: hardware interface
- 1036: user interface
- 1038: memory
- 1040: machine executable instructions
- 1042: pulse sequence commands
- 1044: k-space data
- 1046: magnetic resonance image

## Claims

1. A magnetic resonance breast coil assembly (100, 200), comprising:
- a breast specific medial support (104), wherein the breast specific medial support comprises a first cupped surface (106) configured for providing medial support and vertical support to a breast (102), and a
- a breast specific tiltable flap (108), wherein the breast specific tiltable flap comprises a second cupped surface (110) configured for providing lateral support and vertical support to the breast, wherein the breast specific tiltable flap has an adjustable tilt (608, 700) configured to at least partially stabilize the breast by tilting to hold the breast between the first cupped surface and the second cupped surface; and
- at least one breast specific magnetic resonance imaging coil (402, 404, 406, 408).

2. The magnetic resonance breast coil assembly of claim 1, wherein the breast specific tiltable flap is configured for tilting away from the breast specific medial support to provide an access port (300) to the breast.

3. The magnetic resonance breast coil assembly of claim 1 or 2, wherein the adjustable tilt is implemented by supporting the breast specific tiltable flap from a pivot shaft with a self-locking interference fit (700).

4. The magnetic resonance breast coil assembly of claim 1 or 2, wherein the adjustable tilt is implemented by supporting the breast specific tiltable flap from a pivot shaft with a ratchet mechanism (608).

5. The magnetic resonance breast coil assembly of claim 3 or 4, wherein the magnetic resonance breast coil assembly further comprises radio frequency cable (802) routed through the pivot shaft for connecting to a portion of the at least one breast specific magnetic resonance imaging coil.

6. The magnetic resonance breast coil assembly of any one of the preceding claims, wherein the at least one breast specific magnetic resonance imaging coil comprises one or more medial breast surface coils (402) located adjacent to the first cupped surface, wherein the at least one breast specific magnetic resonance imaging coil preferably comprises at least two medial breast surface coils located adjacent to the first cupped surface, wherein the at least one breast specific magnetic resonance imaging coil more preferably comprises at least four medial breast surface coils located adjacent to the first cupped surface.

7. The magnetic resonance breast coil assembly of any one of the preceding claims, wherein the at least one breast specific magnetic resonance imaging coil comprises one or more lateral breast surface coils (404) located adjacent to the second cupped surface, wherein the at least one breast specific magnetic resonance imaging coil preferably comprises at least two lateral breast surface coils located adjacent to the second cupped surface, wherein the at least one breast specific magnetic resonance imaging coil more preferably comprises at least four lateral breast surface coils located adjacent to the second cupped surface.

8. The magnetic resonance breast coil assembly of claim 7, wherein the one or more lateral breast surface coils are located on the second cupped surface.

9. The magnetic resonance breast coil assembly of any one of the preceding claims, wherein the at least one breast specific magnetic resonance imaging coil comprises at least one anterior surface coil (406), and wherein the at least one anterior surface coil is preferably configured for imaging an axial region of the subject.

10. The magnetic resonance breast coil assembly of any one of the preceding claims, wherein the at least one breast specific magnetic resonance imaging coil comprises at least one posterior surface coil (408), and wherein the at least one posterior surface coil is preferably distal to the breast.

11. The magnetic resonance breast coil assembly of any one of the preceding claims, wherein the breast specific tiltable flap is configured for a translational motion (116) in a lateral direction, wherein the translational motion is controlled using any one of the following: a releasable ratchet (600) and a cylindrical cam (504).

12. The magnetic resonance breast coil assembly of any one of the preceding claims, wherein the magnetic resonance breast coil assembly further comprises a subject support (202) configured for supporting a subject (400) in a horizontal position, wherein the subject support comprises at least one breast opening (208) for receiving the breast of the subject; wherein the subject support is preferably formed from a flexible surface (206) supported by a frame (204), wherein the flexible surface is preferably formed from a textile, wherein the subject support is preferably configured such that a chest region of the subject is supported horizontally and a pelvic region of the subject is inclined below the chest region.

13. A magnetic resonance imaging system comprising the magnetic resonance breast coil assembly of any one of claims 1 through 12.

14. A method of magnetic resonance imaging using the magnetic resonance breast coil assembly of any one of claims 1 through 12, wherein the method comprises:
- stabilizing (1100) the breast by the breast specific adjustable tilt flap with that the first cupped surface and the second cup surface;
- acquiring (1102) by a magnetic resonance imaging (1000) system k-space data (1044) using the at least one breast specific magnetic resonance imaging coil; and
- reconstructing (1104) a magnetic resonance image (1046) using the k-space data.

15. The method of claim 14, wherein the breast specific tiltable flap is configured for tilting away from the breast specific medial support to provide an access port (300) to the breast, wherein the method further comprises:
- adjusting the breast specific tiltable flap to provide the access port to the breast; and
- adjusting a position of the breast before adjusting the adjustable tilt such that the first cupped surface and the second cupped surface stabilize the breast.
